# EUROPÄISCHE PATENTANMELDUNG

(11) **EP 0 903 332 A1**
(43) Veröffentlichungstag der Anmeldung: **24.03.1999**
(21) Anmeldenummer: 98115966.8
(22) Anmeldetag: 23.03.1995
(51) Int. Cl.: C07C 17/266, C07C 22/04

(54) **Verfahren zur Herstellung von substituierten Phenylpropenen**

(30) Priorität: 05.04.1994 DE 4411667
(62) Teilanmeldung aus: 95104285.2
(71) Anmelder: BAYER AG, 51368 Leverkusen (DE)
(72) Erfinder: Lantzsch, Reinhard, Dr., 42115 Wuppertal (DE); Fuchs, Rainer, Dr., 42113 Wuppertal (DE)

(57) **Zusammenfassung**

Die Erfindung betrifft ein Verfahren zur Herstellung von substituierten Phenylpropenen der Formel (IIa) 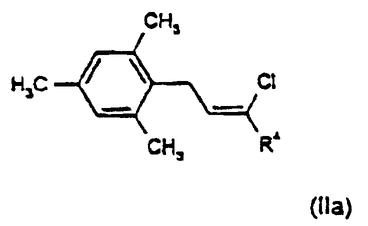 in welcher
- R⁴: für Wasserstoff oder Methyl steht,
dadurch gekennzeichnet, daß man Mesitylen der Formel (VI) 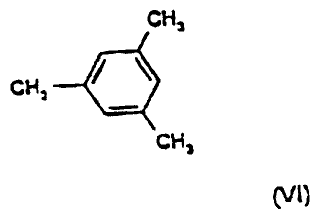 mit Dichloralkenen der Formel (VII) 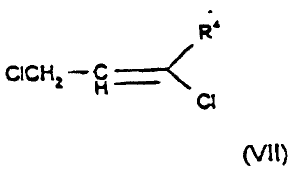 in welcher
- R⁴: für Wasserstoff oder Methyl steht,
gegebenenfalls in Gegenwart eines Verdünnungsmittels und gegebenenfalls in Gegenwart eines Katalysators umsetzt.

## Beschreibung

Die Erfindung betrifft neue Verfahren zur Herstellung von teilweise bekannten Phenylessigsäurederivaten, neue Zwischenprodukte zu ihrer Herstellung und ein Verfahren zu deren Herstellung.

Es ist bekannt, daß man substituierte Phenylessigsäuren und ihre Derivate erhält, wenn man entsprechende substituierte Aromaten durch eine Chlormethylierung oder Brommethylierung in die Chlormethyl- oder Brommethylaromaten überführt, diese dann mit Cyaniden umsetzt und sie anschließend zur Säure verseift (vgl. z.B. J. Org. Chem. 58, Houben-Weyl, Methoden der Organischen Chemie, Georg Thieme Verlag, Stuttgart, Band 5/4 Seite 484, 1960, Houben-Weyl, Methoden der Organischen Chemie, Georg Thieme Verlag, Stuttgart, Band VIII, Seite 427, 1952). Dieses Verfahren weist jedoch den Nachteil auf, daß wegen der Möglichkeit der bei der Halogenmethylierung entstehenden kanzerogenen Bishalogenmethylether ein erhöhter Aufwand an Sicherheitsvorkehrungen bei der Reaktionsdurchführung erforderlich ist.

Weiter ist bekannt, daß man Phenylessigsäuren durch Carboxylierung von Benzylhalogeniden unter Phasentransferbedingungen erhält (Tetrahedron Lett., 24 (37), 4005-4008; J. Chem. Soc., Chem. Commun. (24), 1090-1091). Ein erheblicher Nachteil dieser Verfahren ist neben dem Einsatz von Eisen- und Cobaltcarbonylen, daß sie zum Teil unter Druck durchgeführt werden müssen und zu Reaktionsgemischen führen.

Gegenstand der vorliegenden Erfindung ist
1) ein Verfahren zur Herstellung von Phenylessigsäurederivaten der Formel (I) 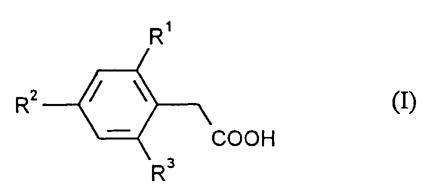 in welcher
   - R¹, R² und R³: jeweils unabhängig voneinander für Wasserstoff, Alkyl oder Alkoxy stehen,
   dadurch kennzeichnet, daß man substituierte Phenylpropene der Formel (II) 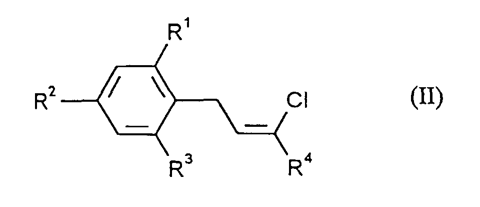 in welcher
   - R¹, R² und R³: die oben angegebene Bedeutung haben und
   - R⁴: für Wasserstoff oder Methyl steht, entweder

   a) in Gegenwart von inerten Lösungsmitteln einer Ozonolyse unterwirft, anschließend die so erhaltenen Aldehyde der Formel (III) 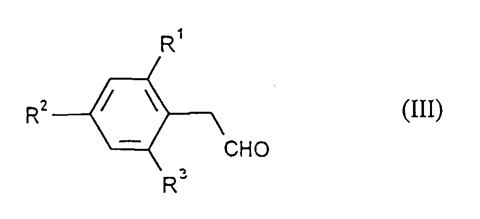 gegebenenfalls isoliert und diese dann in Gegenwart eines Verdünnungsmittels, in Gegenwart einer Säure und in Gegenwart eines Oxidationsmittels zu den Verbindungen der Formel (I) umsetzt,
      oder
   b) in Gegenwart von Alkoholen der Formel (IV)

      R⁵-OH (IV)

      in welcher
      - R⁵: für Alkyl, insbesondere für C₁-C₄-Alkyl und ganz besonders für Methyl, Ethyl oder Butyl steht,
      einer Ozonolyse unterwirft und die so erhaltenen Phenylacetaldehydacetale der Formel (V) 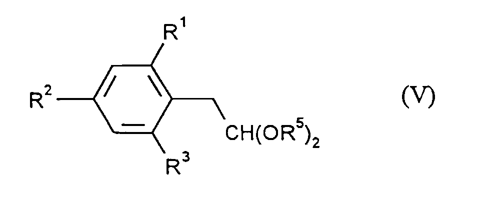 in welcher
      R¹, R², R³ und R⁵ die oben angegebene Bedeutung haben,
         gegebenenfalls isoliert und
         diese anschließend direkt oder nach Hydrolyse zu den Aldehyden der Formel (III), gegebenenfalls in Gegenwart eines Verdünnungsmittels in Gegenwart einer Säure und in Gegenwart eines Oxidationsmittels zu den Verbindungen der Formel (I) umsetzt.
2) Ein Verfahren zur Herstellung von substituierten Phenylpropenen der Formel (IIa) 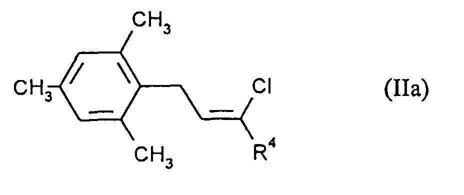 in welcher
   - R⁴: für Wasserstoff oder Methyl steht,
   9697 dadurch gekennzeichnet, daß man Mesitylen der Formel (VI) 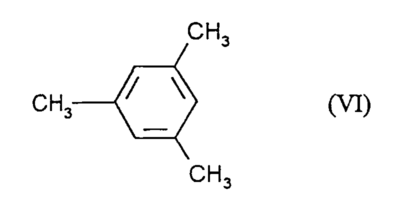 mit Dichloralkenen der Formel (VII) 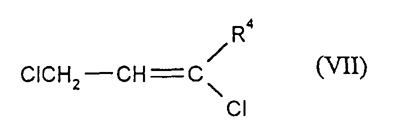 in welcher
   - R⁴: für Wasserstoff oder Methyl steht,
   gegebenenfalls in Gegenwart eines Verdünnungsmittels und gegebenenfalls in Gegenwart eines Katalysators umsetzt.
3) Neue Mesitylacetaldehydacetale der Formel (Va) 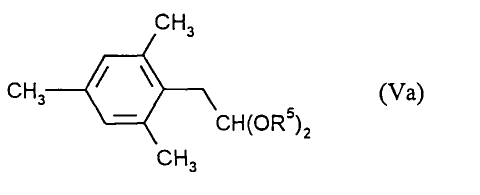 in welcher
   - R⁵: die oben angegebene Bedeutung hat.

Es ist als ausgesprochen überraschend anzusehen, daß nach dem erfindungsgemäßen Verfahren (1) die Phenylessigsäure-Derivate der Formel (I) nahezu quantitativ und in hoher Reinheit durch Ozonolyse und Oxidation erhalten werden, da bekannt ist, daß die sowohl bei Verfahren (1a), als auch bei Verfahren (1b) als Zwischenstufe entstehenden Arylacetaldehyde der Formel (III) sehr instabile Verbindungen sind und leicht mit sich selbst reagieren. Ferner können sich Arylessigsäuren leicht unter CO₂-Abspaltung zersetzen. Schließlich mußte der Fachmann eine Weiteroxidation zur substituierten Benzoesäure erwarten.

Ein weiterer Vorteil des erfindungsgemäßen Verfahrens ist, daß die Bildung des stark kanzerogenen Bishalogenmethylethers, der anfällt, wenn Phenylessigsäurederivate über die Zwischenstufe der Chlormethylierung von Aromaten hergestellt werden, vermieden wird. Das neue Verfahren kann somit unter deutlich verbesserten Sicherheits- und Umweltaspekten durchgeführt werden.

Nach dem erfindungsgemäßen Verfahren werden bevorzugt Verbindungen der Formel (I) hergestellt, in welcher
- R¹, R² und R³: jeweils unabhängig voneinander für Wasserstoff, C₁-C₄-Alkyl oder C₁-C₄-Alkoxy stehen.

Nach dem erfindungsgemäßen Verfahren werden besonders bevorzugt Verbindungen der Formel (I) hergestellt, in welcher
- R¹, R² und R³: jeweils unabhängig voneinander für Wasserstoff, Methyl, Ethyl, Methoxy oder Ethoxy stehen.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, als auch zwischen den jeweiligen Bereichen und Vorzugsbereichen, beliebig kombiniert werden.

Verwendet man beispielsweise für die erste Stufe 1-Chlor-3-(2,4,6-trimethylphenyl)-1-propen als Ausgangsstoff, Methylenchlorid als Lösungsmittel und Ozon und für die zweite Stufe Essigsäure und Wasserstoffperoxid, so läßt sich das erfindungsgemaße Verfahren (1a) durch das folgende Formelschema beschreiben:

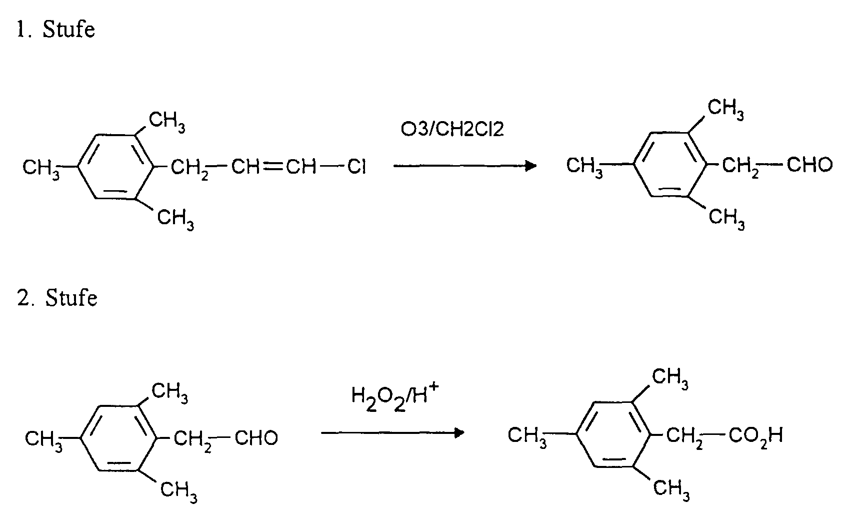

Verwendet man beispielsweise für die erste Stufe 1-Chlor-3-(2,4,6-trimethylphenyl)-1-propen und Methanol und Ozon als Ausgangsstoffe und für die zweite Stufe Essigsäure und Wasserstoffperoxid, so laßt sich das erfindungsgemäße Verfahren (1b) durch das folgende Formelschema beschreiben:

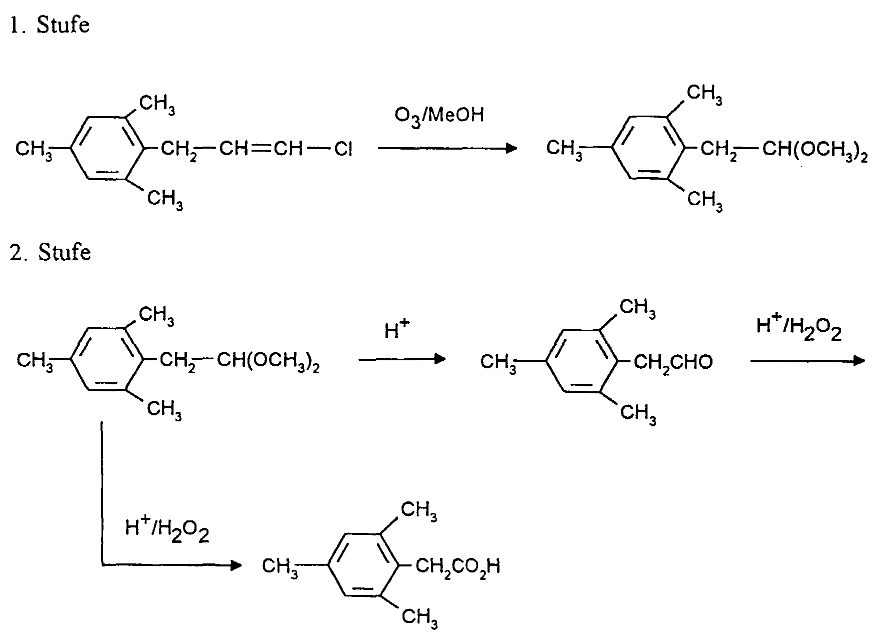

Die bei dem oben unter (1) angegebenen erfindungsgemäßen Verfahren zur Herstellung der Verbindungen der Formel (I) als Ausgangsstoffe zu verwendenden substituierten Phenylpropene sind durch die Formel (II) allgemein definiert. In der Formel (II) stehen R¹, R² und R³ vorzugsweise bzw. insbesondere für diejenige Bedeutung, die bereits oben im Zusammenhang mit der Beschreibung der Verbindungen der Formel (I) als bevorzugt bzw. insbesondere bevorzugt für R¹, R² und R³ angegeben wurde. R⁴ steht für Wasserstoff oder Methyl.

Die Verbindungen der Formel (II) sind bekannt oder können nach bekannten Verfahren hergestellt werden (vgl. Comptes Rendus 213, 619-620 (1941)). Die bekannten und die noch nicht bekannten Verbindungen der Formel (II) können beispielsweise nach dem unter (2) beschriebenen erfindungsgemäßen Verfahren erhalten werden.

Die bei dem oben unter (1b) angegebenen Verfahren außerdem als Ausgangsstoffe zu verwendenden Alkohole sind durch die Formel (IV) allgemein definiert. In dieser Formel (IV) hat R⁵ bevorzugt bzw. besonders bevorzugt diejenige Bedeutung, die bereits oben im Zusammenhang mit der Beschreibung der Verbindungen der Formel (IV) als bevorzugt bzw. besonders bevorzugt für R⁵ angegeben wurde.

Die Alkohole der Formel (IV) sind allgemein bekannte Verbindungen der organischen Chemie.

Die bei dem oben unter (1b) angegebenen Verfahren als Zwischenprodukte erhältlichen Phenylacetaldehydacetale sind durch die Formel (V) allgemein definiert. In dieser Formel (V) stehen R¹, R², R³ und R⁵ vorzugsweise bzw. insbesondere für diejenige Bedeutung, die bereits oben im Zusammenhang mit der Beschreibung der Verbindungen der Formeln (I) und (IV) als bevorzugt bzw. insbesondere bevorzugt für R¹, R², R³ und R⁵ angegeben wurde.

Einige der Phenylacetaldehydacetale der Formel (V) sind bereits prinzipiell aus der Literatur bekannt (vgl. z.B. EP 403 841, FR 2 577 920). Die bekannten und die noch nicht bekannten Phenylacetaldehydacetale der Formel (V) können nach dem unter (1b) beschriebenen erfindungsgemäßen Verfahren erhalten werden.

Die bei dem oben unter (1) angegebenen Verfahren weiterhin als Zwischenprodukte erhältlichen Aldehyde der Formel (III) sind allgemein bekannte Verbindungen der organischen Chemie.

Das unter (1a) beschriebene erfindungsgemäße Verfahren wird vorzugsweise in Gegenwart eines Verdünnungsmittels durchgeführt.

Als Verdünnungsmittel zur Durchführung des unter (1a) beschriebenen Verfahrens kommen alle unter den Reaktionsbedingungen inerten, üblichen organischen Lösungsmittel in Betracht. Hierzu gehören beispielsweise insbesondere inerte Solventien: beispielsweise Chlorkohlenwasserstoffe wie Methylenchlorid, Ketone, wie Aceton, Ester wie Essigsäuremethyl- oder -ethylester und Kohlenwasserstoffe wie Pentan, Hexan oder Cyclohexan.

Die Reaktionstemperaturen können bei der Durchführung der unter (1a) und (1b) beschriebenen erfindungsgemäßen Verfahren in einem größeren Bereich variiert werden. Im allgemeinen arbeitet man bei Temperaturen zwischen -70°C und +20°C, vorzugsweise zwischen -30°C und 0°C.

Das unter (1b) beschriebene erfindungsgemäße Verfahren wird vorzugsweise in Gegenwart von Alkoholen durchgeführt. Als Alkohole zur Durchführung des unter (1b) beschriebenen Verfahrens kommen primäre Alkohole, insbesondere Methanol, Ethanol oder n-Butanol in Betracht.

Das unter (1a) beschriebene erfindungsgemäße Verfahren wird so durchgeführt, daß das entsprechende substituierte Phenylpropen der Formel (II) in einem der oben angegebenen Lösungsmittel vorgelegt wird und danach bei der angegebenen Temperatur so lange Ozon eingeleitet wird, bis die Reaktion beendet ist. Das überschüssige Ozon wird mit einem Inertgas, wie beispielsweise Stickstoff oder Argon ausgeblasen oder mit einem Reduktionsmittel, wie beispielsweise Dimethylsulfid oder Tetrahydrothiophen zerstört, wobei gleichzeitig das gebildete Ozonid gespalten wird. Statt Sulfiden können auch andere Reduktionsmittel verwendet werden, wie beispielsweise Thioharnstoff, Natriumhydrogensulfitlösung, Zinkstaub in Essigsäure. Die Spaltung gelingt auch durch Wasserstoff mittels katalytischer Hydrierung nach allgemein bekannten Methoden. Ferner ist die Verwendung von Aminen, wie beispielsweise Triethylamin, zur Spaltung des Primärozonides möglich.

Das unter (1b) beschriebene erfindungsgemäße Verfahren wird analog dem oben unter (1a) beschriebenen Verfahren, jedoch in Gegenwart von Alkoholen als Lösungsmittel durchgeführt. Man erhält dann die Phenylacetaldehydacetale der Formel (V), welche nach üblichen Methoden, beispielsweise durch saure Hydrolyse in die Aldehyde der Formel (III) übergeführt werden können (siehe Linke in Houben-Weyl, Band E3, Aldehyde, Seite 362 - 367, 1983).

Es kann gegebenenfalls auch vorteilhaft sein, die nach dem erfindungsgemäßen Verfahren (1a) und (1b) entstandenen Aldehyde der Formel (III) und die Phenylacetaldehydacetale der Formel (V) nicht zu isolieren, sondern nach dem Abdestillieren des Lösungsmittels in einer Eintopfreaktion mit Wasserstoffperoxid-Lösung, vorzugsweise unter Zusatz von Säuren, wie beispielsweise Essigsäure oder Propionsäure, direkt zu den Phenylessigsäuren der Formel (I) weiter umzusetzen.

Die Phenylessigsäuren werden durch Extraktion der wäßrigen Phase oder durch Abfiltrieren erhalten.

Bei dem oben unter (2) angegebenen Verfahren wird Mesitylen der Formel (VI) als Ausgangsstoff verwendet.

Die bei dem oben unter (2) angegebenen Verfahren als Ausgangsstoffe außerdem zu verwendenden Dichloralkene der Formel (VII) sind ebenfalls bekannte Synthesechemikalien.

Verwendet man beispielsweise Mesitylen und 1,3-Dichlorpropen als Ausgangsstoffe und Aluminiumchlorid als Katalysator, so laßt sich das erfindungsgemäße Verfahren (2) durch das folgende Formelschema beschreiben:

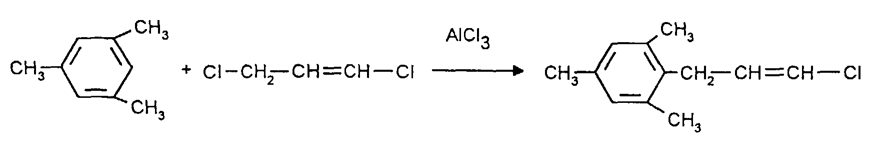

Es ist als ausgesprochen überraschend anzusehen, daß nach dem erfindungsgemäßen Verfahren (2) die Friedel-Crafts-Alkylierung von Mesitylen mit 1,3-Dichlorpropen oder Dichlorbuten mit sehr guten Ausbeuten von ca. 90% der Theorie verläuft, da in der Literatur nur Ausbeuten von 10% beschrieben werden (Compt. Rendus 213, 619, 1941). Vielmehr war eine Addition an die Doppelbindung (Chem. Berichte 66, 1100) oder eine Umlagerung der Methylgruppen im Mesitylen unter dem Einfluß von Lewis-Säuren zu erwarten.

Das erfindungsgemäße Verfahren stellt somit eine wertvolle Bereicherung des Standes der Technik dar.

Das unter (2) beschriebene erfindungsgemäße Verfahren kann in Gegenwart eines Verdünnungsmittels durchgeführt werden:

Als Verdünnungsmittel zur Durchführung des unter (2) beschriebenen Verfahrens kommen alle unter den Reaktionsbedingungen inerten, üblichen organischen Lösungsmittel in Betracht. Hierzu gehören beispielsweise insbesondere inerte Solventien wie Pentan, Hexan, Cyclopentan, Cyclohexan, Nitroalkane, Schwefelkohlenstoff; Chlorkohlenwasserstoffe, wie Methylenchlorid, Dichlorethan oder Tetrachlorethan, Nitrile, wie Acetonitril, Ether, wie Diethylether, Diisopropylether, Methyl-tert.-Butylether, TAME, Dimethoxyethan oder Amide, wie Dimethylformamid.

Die Reaktionstemperaturen können bei der Durchführung des unter (2) beschriebenen erfindungsgemäßen Verfahrens in einem größeren Bereich variiert werden. Im allgemeinen arbeitet man bei Temperaturen zwischen -30°C und +50°C, vorzugsweise bei Temperaturen zwischen -10°C und +30°C.

Das unter (2) beschriebene erfindungsgemäße Verfahren wird im allgemeinen unter Normaldruck durchgeführt. Es ist jedoch auch möglich, unter erhöhtem oder vermindertem Druck zu arbeiten.

Die unter (2) beschriebene erfindungsgemäße Verfahrensstufe wird in Gegenwart von starken Säuren durchgeführt. Als Beispiele für solche Säuren seien genannt: Lewissäuren, wie beispielsweise Aluminiumchlorid, Aluminiumbromid, Eisen(III)chlorid, Zinntetrachlorid, Zinkchlorid, Titantetrachlorid oder Bortrifluorid; Mineralsäuren, wie beispielsweise Schwefelsäure oder Salzsäure, welche gegebenenfalls auch gasförmig eingesetzt werden kann und Sulfonsäuren, wie beispielsweise p-Toluolsulfonsäure oder Trifluormethansulfonsäure. Bevorzugt sind Aluminiumchlorid und Eisen(III)chlorid.

Auf beispielsweise 1 Mol der Verbindungen der Formel (VII) werden 0,01-30,0 Mol%, bevorzugt 0,05 - 10,0 Mol% Lewissäure, Mineralsäure oder Sulfonsäure und 1 bis 20 Mol Mesitylen in 50-500 ml in einem der oben genannten Verdünnungsmittel eingesetzt.

In einer bevorzugten Ausführungsform wird das unter (2) beschriebene erfindungsgemäße Verfahren so durchgeführt, daß das Mesitylen der Formel (VI) selbst als Lösungsmittel oder in einem der oben angegebenen Lösungsmittel mit einer Lewissäure, wie beispielsweise Aluminiumchlorid, vorgelegt wird, danach mit dem Dichloralken der Formel (VII) versetzt wird und danach bei der angegebenen Temperatur so lange gerührt wird, bis die Reaktion beendet ist.

In einer weiteren Ausführungsform wird das unter (2) beschriebene erfindungsgemäße Verfahren durchgeführt, indem man die Dichloralkene der Formel (VII) und das Mesitylen der Formel (VI) gegebenenfalls in einem Lösungsmittel vorlegt, danach mit einer Lewissäure oder Mineralsäure oder Sulfonsäure versetzt und bei der angegebenen Temperatur bis zum Reaktionsende nachrührt.

Die Aufarbeitung kann nach üblichen Methoden erfolgen, beispielsweise durch Verdünnen des Reaktionsansatzes mit Wasser, Extraktion mit einem organischen, mit Wasser praktisch nicht mischbaren Lösungsmittel, Trocknen der organischen Phase und Entfernen des Lösungsmittels unter vermindertem Druck. Das so erhaltene Rohprodukt kann durch Destillation gereinigt werden.

Die unter (3) angegebenen Mesitylacetaldehyde sind durch die Formel (Va) allgemein definiert.
- R⁵: steht bevorzugt für C₁-C₄-Alkyl, besonders bevorzugt für Methyl, Ethyl und n-Butyl.

Die Mesitylacetaldehydacetale der Formel (Va) sind neu und Gegenstand der Erfindung. Sie können nach dem erfindungsgemäßen Verfahren (1b) erhalten werden.

Die nach dem erfindungsgemäßen Verfahren (1) herzustellenden Phenylessigsäurederivate der Formel (I) und die nach dem erfindungsgemäßen Verfahren (2) herzustellenden, substituierten Phenylpropene der Formel (IIa) können als Ausgangsstoffe zur Herstellung von Schädlingsbekämpfungsmitteln verwendet werden (vgl. z.B. EP-A 528 156).

Die Erfindung soll durch die folgenden Beispiele verdeutlicht werden:

### Beispiele

### Beispiel 1

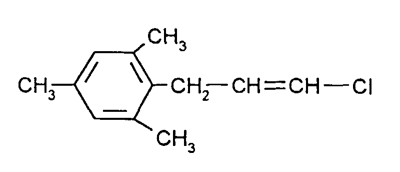

### 1 Chlor-3-(2,4,6-trimethylphenyl)-prop-1-en

30g (0,25 Mol) Mesitylen und 0,7g Aluminiumchlorid werden vorgelegt. Dann tropft man bei 20 - 25°C 5,55g (0,05 Mol) 1,3-Dichlorpropen (cis/trans) unter Kühlung zu.

Nach beendeter Zugabe rührt man noch eine Stunde bei 25°C nach, gießt dann auf 100ml Eiswasser und trennt die organische Phase ab. Die wäßrige Phase wird noch zweimal mit Toluol nachextrahiert. Die vereinigten organischen Phasen werden getrocknet und Toluol sowie überschüssiges Mesitylen unter vermindertem Druck abdestilliert.

Man erhält 12,2g 1-Chlor-3-(2,4,6-trimethylphenyl)-prop-1-en (Gehalt: 78,0%, GC).
Nach Destillation im Kugelrohr erhält man 8,7g (Gehalt: GC: 96,9%) entsprechend 87% der Theorie vom Siedepunkt Kp = 95 - 97°C / 1mbar.

### Beispiel 2

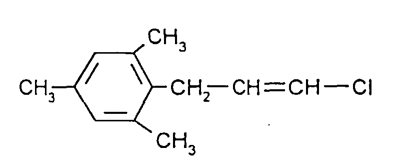

60g (0,5 Mol) Mesitylen und 0,4g (0,0025 Mol) Eisen(III)chlorid werden vorgelegt und ca. 15 Minuten gerührt. Dann werden 11,1g (0,1 Mol) 1,3-Dichlorpropen bei 20 - 25°C zugetropft Nach vollständiger Zugabe wird noch ca. 30 Minuten nachgerührt, bis die Gasentwicklung beendet ist. Dann wird noch 15 Minuten bei 30°C nachgerührt. Die Reaktionslösung wird in 250ml Eiswasser eingerührt, die organische Phase abgetrennt und mit Wasser neutral gewaschen. Nach Destillation (bei ca. 2mbar) erhält man 15,9g 1-Chlor-3-(2,4,6-trimethylphenyl)-prop-1-en, was einer Ausbeute von 81,7% der Theorie entspricht.

### Beispiel 3

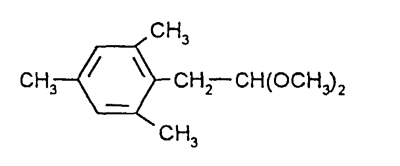

1,95g (0,01 Mol) 1-Chlor-3-(2,4,6-trimethylphenyl)-prop-1-en werden in 60ml Methanol gelöst und auf -30°C abgekühlt. Danach wird mittels eines Ozongenerators erzeugtes Ozon innerhalb von 40 Minuten durch das Reaktionsgemisch geleitet, wobei sich die klare, farblose Lösung leicht grau färbt. Der Fortgang der Oxidation wird durch Dünnschicht- oder Gaschromatographie verfolgt. Nach vollständigem Umsatz wird das gebildete Ozonid mit Dimethylsulfid gespalten und das Methanol im Vakuum abdestilliert. Man erhält praktisch quantitativ das Dimethylacetal des 2,4,6-Trimethylphenylacetaldehyds als Rohprodukt, welches durch Destillation im Kugelrohr (2mbar, Manteltemperatur 80 - 100°C) weiter gereinigt werden kann, Abtrennung des aus Dimethylsulfid gebildeten Dimethylsulfoxids.

Das Produkt wurde durch das Massenspektrum charakterisiert: ^{m}/ₑ = 208 (Molpeak), 177, 147, 133, 75 (basepeak), 47.

### Beispiel 4

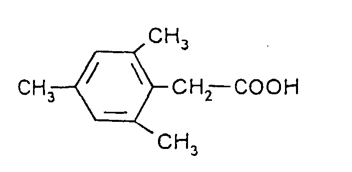

Das nach Beispiel 3 erhaltene Rohprodukt des Dimethylacetals des 2,4,6-Trimethylphenylacetaldehyds wird mit 3,5ml Wasser, 4ml Essigsäure und 2ml 30%igem Wasserstoffperoxid versetzt und bei 25 - 35°C 12 Stunden gerührt. Nach Eindampfen bis zur Trockne wird in Wasser/Ethanol aufgenommen. Dann filtriert man ab und trocknet.

Man erhält 1,45g (81% der Theorie) 2,4,6-Trimethylphenylessigsäure vom Schmelzpunkt 165°C.

### Beispiel 5

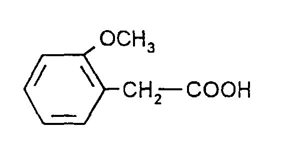

2g (0,01 Mol) 2-Chlor-3-(2'-methoxy-phenyl)-2-buten (bekannt aus C. A. 73 (13), 66 192 c) werden in 60ml Methylenchlorid gelöst und auf -50°C abgekühlt. Danach wird mittels eines Ozongenerators erzeugten Ozon innerhalb von 45 Minuten durch das Reaktionsgemisch geleitet. Nach beendeter Reaktion wird überschüssiges Ozon mit Stickstoff ausgetrieben und das gebildete Ozonid mit 2 g (0,02 Mol) Triethylamin versetzt. Man läßt auf Raumtemperatur kommen, wäscht die organische Phase mit Wasser und dampft ein. Der Rückstand wird mit 5 ml Eisessig und bei 60°C mit 3ml 30%iger Wasserstoffperoxidlösung versetzt und 2 Stunden gerührt. Nach dem Abkühlen wird mit Wasser verdünnt und dreimal mit Methylenchlorid extrahiert.

Nach dem Trocknen und Abdestillieren erhält man 1,5 g (90% der Theorie) 2-Methoxyphenylessigsäure vom Schmelzpunkt 123°C.

## Patentansprüche

1. Verfahren zur Herstellung von substituierten Phenylpropenen der Formel (IIa) 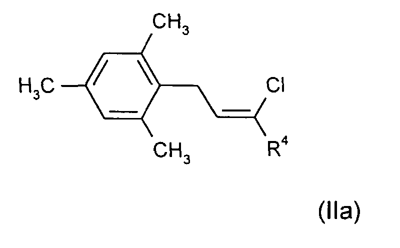 in welcher
R⁴ für Wasserstoff oder Methyl steht,
dadurch gekennzeichnet, daß man Mesitylen der Formel (VI) 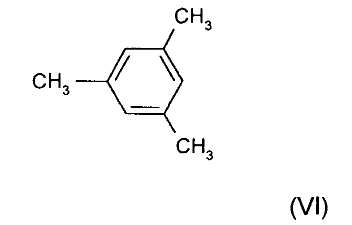 mit Dichloralkenen der Formel (VII) 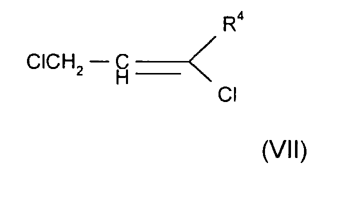 in welcher
R⁴ für Wasserstoff oder Methyl steht,
gegebenenfalls in Gegenwart eines Verdünnungsmittels und gegebenenfalls in Gegenwart eines Katalysators umsetzt.
